# FASCICULE DE BREVET EUROPEEN

(11) **EP 1 397 124 B1**
(45) Date de publication et mention de la délivrance du brevet: **29.11.2006**
(21) Numéro de dépôt: 02748965.7
(22) Date de dépôt: 19.06.2002
(51) Int. Cl.: A61K 31/64, A61P 17/02

(54) **UTILISATION D'AGENTS ANTIDIABETIQUES POUR FABRIQUER UN MEDICAMENT AYANT UN EFFET CICATRISANT**
VERWENDUNG VON ANTIDIABETIKA ZUR HERSTELLUNG EINES VERNARBUNGSFÖRDERNDEN ARZNEIMITTELS
USE OF ANTIDIABETICS FOR MAKING A MEDICINE WITH CICATRIZING EFFECT

(30) Priorité: 20.06.2001 FR 0108130; 20.06.2001 US 299196 P
(43) Date de publication de la demande: 17.03.2004
(73) Titulaire: Merck Sante, 69008 Lyon (FR)
(72) Inventeur: BRIET, Philippe, F-69003 Lyon (FR)
(74) Mandataire: Bernasconi, Jean Raymond
(86) Numéro de dépôt international: PCT/FR2002/002124
(87) Numéro de publication internationale: WO 2003/003971

(56) Documents cités:
- WO-A-01/21159
- WO-A-01/76650
- WO-A-93/08815
- WO-A-98/32429
- WO-A-98/58658
- WO-A2-01/26639
- WO-A2-02/15892
- FR-A- 2 213 778
- FR-A- 2 802 814
- US-A- 3 072 527
- US-A- 4 131 652
- US-A- 5 145 679
- MASCARDO R.N. ET AL: "Glyburide modulates insulin-mediated locomotor response of confluent cell cultures to wounding." DIABETES, (1987) 36/12 (1476-1482)., XP008000808
- CAVALERI F ET AL: "OSSERVAZIONI SULL'IMPIEGO DEGLI IPOGLICEMIZZANTI ORALI IN DERMOPAZIENTI DIABETICI, CON PARTICOLARE RIGUARDO ALL'ASSOCIAZIONE GLIBENCLAMIDE-FENFORMINA (SUGUAN(R))" GIORNALE ITALIANO DI DERMATOLOGIA E VENEREOLOGIA, TURIN, IT, vol. 112, no. 2, mars 1977 (1977-03), pages 255-266, XP000981768 ISSN: 0392-0488
- DATABASE WPI Week 200062, 2 août 2000 (2000-08-02) Derwent Publications Ltd., London, GB; AN 2000-641024 XP002192081 TAKAHASHI ET AL.: "Phosphoric acid derivative" & JP 2000 212191 A (SANKYO CO LTD), 2 août 2000 (2000-08-02)
- VERGER M F ET AL: "L'INSULINOTHERAPIE CONTINUE AMELIORE LA CICATRISATION DES PLAIES DES PIEDS DIABETIQUES" REVUE DE MEDECINE INTERNE, CMR, ASNIERES, FR, vol. 7, no. 2, mars 1986 (1986-03), pages 127-132, XP000981769 ISSN: 0248-8663
- MIHAILOVA E V ET AL: "GOOD HEALING EFFECTS OF DI-METHYL SULPHOXIDE AND INSULIN ON NECROBIOSIS LIPODICA DIABETICORUM IN DYPE-1 DIABETES" EUROPEAN JOURNAL OF ENDOCRINOLOGY, SCANDINAVIAN UNIVERSITY PRESS, NO, vol. 130, no. SUPPL 2, 1994, page 202 XP000981777 ISSN: 0804-4643
- O'NEIL M.J. ET AL: 'The Merck Index Thirteenth Edition', 2001, MERCK & CO., INC., WHITEHOUSE STATION, N.J. * page 1696 *

## Description

La présente invention concerne la cicatrisation des plaies.

Elle concerne en particulier l'utilisation d'agents antidiabétiques pour fabriquer un médicament ayant un effet cicatrisant.

La cicatrisation des plaies ou des dommages apparentés de différents types de tissus dépend généralement de la prolifération de nouveaux tissus épithéliaux, endothéliaux et conjonctifs. Elle met donc en jeu une série d'évènements coordonnés, cellulaires et moléculaires. Elle peut être retardée ou modifiée par des perturbations métaboliques qui accompagnent certaines maladies de longue durée telles qu'insuffisance veineuse, artérite, diabète et même par certaines thérapies.

Le marché pharmaceutique offre actuellement de nombreuses préparations topiques recommandées pour la cicatrisation des plaies. En fait, leur action résulte de la complémentarité des différents produits qui les composent et qui leur confèrent dans une certaine limite, leur propriété cicatrisante. Elles protègent les plaies du milieu environnant par un pansement antiseptique. Elles stimulent le développement de la vascularisation et régulent l'épidermisation. Ces formes topiques sont constituées principalement d'un mélange lipidique (lanoline, vaseline, glycérine...) dans lequel sont ajoutés des acides (salicylique, benzoïque, malique), des minéraux (oxyde de zinc, de titane) ou des halogénures (iodure d'amidon).

Certaines contiennent également du collagène, du fibrinogène, du protéolysat enzymatique de sérum (apport d'acides aminés) ou encore des vitamines (vitamine A) ou des hormones (acétate de choro-4-testostérone). Il existe également une pommade (Madécasol® tulgras des Laboratoires SYNTEX) dont l'action cicatrisante est apportée par l'addition d'un mélange de trois triterpènes extraits de racines de la plante *Centella asiatica* (TCEA). Ces composés exercent leur propriété en stimulant la biosynthèse du collagène et de glycoaminoglycannes. Cependant, ces extraits peuvent provoquer également chez les patients des allergies de contact.

Il est connu qu'une des complications du diabète réside dans l'apparition d'affections cutanées telles que les ulcères (voir angiodermites nécrotiques ulcéreuses) ou de dermatoses perforantes que les médicaments classiques utilisés lors des traitements du diabète ne parviennent ni à contrôler ni à soigner.

Mascardo et al (Diabetes 36/12, 1476-1482, 1987) divulgue que la gliburide (glibenclamide) administrée oralement facilite la rétractation des lésions induites par l'insuline.

WO 98/32429 décrit l'utilisation de modulateurs de l'ATP endogène pour la guérison de lésions.

US 3,072,527 décrit que l'administration orale de la tolbutamide diminue les lésions d'acné.

WO 98/58658 décrit que les complications associées au diabète, comme la guérison altérée de lésions, peuvent être traitées avec une combinaison d'un agent inhibant la glycosylation et d'un agent modulant le glucose.

Or, les inventeurs de la présente invention ont mis en évidence de façon surprenante que certains agents antidiabétiques possèdent également de fortes propriétés cicatrisantes, c'est à dire une activité stimulante vis-à-vis d'un phénomène physiologique complexe caractérisé entre autre par une croissance cellulaire accrue au niveau de la plaie. Cette prolifération transitoire se produit en réponse à la perte de l'intégrité cutanée et assure la réparation de tissu profond et la reconstitution de l'épiderme au niveau des plaies.

Ainsi, l'application topique d'un de ces composés sous forme de pommade induit une guérison rapide et de longue durée d'ulcères de jambe chez des sujets diabétiques et la répétition de l'application topique du principe actif renforce cet effet. De plus, ces agents antidiabétiques peuvent accélérer également la cicatrisation des plaies atones chez des sujets non diabétiques.

Compte tenu des difficultés rencontrées pour maîtriser la qualité des produits naturels cicatrisants et du nombre d'étapes fastidieuses nécessaires pour isoler ces composés, les agents antidiabétiques oraux, dont la synthèse est en général simple, totale et rapide, se présentent comme des principes actifs très intéressants.

La présente invention concerne donc un ou plusieurs agent(s) antidiabétique(s) choisi(s) parmi le carbutamide, le gliquidone, le glisentide, le glisolamide, le glisoxepide, le glycyclamide, et le glibornuride, pour fabriquer un médicament destiné à améliorer la cicatrisation de plaies ou de lésions par voie topique le(s)dit(s) agent(s) antidiabétique(s) étant le(s) seul(s) principe(s) actif(s) dans le médicament.

Avantageusement, ce médicament est destiné à être appliqué sur la peau.

Ces agents antidiabétiques peuvent être utilisés seuls ou en combinaison.

Par le terme "de sel pharmaceutiquement acceptable", on entend au sens de la présente invention tout sel préparé à partir de tout acide non toxique pharmaceutiquement acceptable, y compris les acides organiques et inorganiques. De tels acides incluent l'acide acétique, benzénesulfonique, benzoïque, citrique, éthanesulfonique, fumarique, gluconique, glutamique, bromhydrique, chlorhydrique, lactique, maléique, malique, mandélique, méthanesulfonique, mucique, nitrique, pamoïque, pantothénique, phosphorique, succinique, tartarique et paratoluènesulfonique. Avantageusement, on utilise l'acide chlorhydrique.

Par le terme de "forme pharmaceutique à usage topique", on entend au sens de la présente invention toute forme pharmaceutique destinée à être appliquée sur la surface de la plaie, en particulier sur la peau ou les muqueuses extérieures ou intérieures, et qui agit localement. En particulier, le médicament peut se présenter sous une forme du type huile, crème, mousse, liniment, lotion, pommade, liquide, gel, lait, poudre ou spray. Les formes peuvent être à véhicule monophasique constituées d'un gel neutre d'hydroxypropylcellulose ou d'un gel chargé formé de carboxyméthylcellulose de sodium. On peut également préparer des crèmes, formes à véhicule biphasique, comportant une phase hydrophile dispersée dans une phase lipophile. De préférence, le médicament n'est pas sous la forme d'un pansement à base de polymère.

Dans un exemple particulier, le médicament contient de 0,02 à 2 % en poids d'un de ces agents antibiotiques ou de son sel pharmaceutiquement acceptable et un excipient approprié.

Ces excipients peuvent être choisis parmi des composés présentant une bonne compatibilité avec ces principes actifs. Il s'agit par exemple des polymères hydrosolubles de type polymère naturel, tels les polysaccharides (gomme xanthane, gomme de caroube, peptine ...) ou polypeptides, des dérivés cellulosiques type méthylcellulose, hydroxypropylcellulose, hydroxypropyl-méthylcellulose ou encore des polymères synthétiques, polaxamers, carbomers, PVA ou PVP.

Enfin, il est à la portée de tout homme de l'art d'ajouter dans ces médicaments divers excipients par exemple des cosolvants comme l'éthanol, le glycérol, l'alcool benzylique, des humectants (glycérol), des agents facilitant la diffusion (transcurol, urée), ou encore des conservateurs anti-bactériens (p-hydroxybenzoate de méthyle à 0,15 %).

Les agents antidiabétiques utilisés dans la présente invention peuvent donc améliorer la cicatrisation de tout type de plaies ou lésions. Ces plaies ou lésions peuvent -être du type incisions chirurgicales, brûlures thermiques, chimiques ou provoquées par une irradiation, abrasions, lacérations, amputations, ulcères ischémiques ou de décubitus, lésions ou ulcères de la bouche ou les lésions de la cornée, et en particulier celles occasionnées par une intervention chirurgicale réalisée chez des sujets diminués, âgés, traités par radio ou chimiothérapie, ou diabétiques. Il en est de même de l'ensemble des dermatoses observées chez les malades dont la circulation cutanée est déficiente (lésions érythémateuses, vascularites) et de l'ensemble des plaies observées chez les sujets diabétiques. Les compositions pharmaceutiques et médicaments selon l'invention apparaissent comme bénéfiques également dans la thérapie des nécroses tissulaires, post-thrombotiques par exemple.

## Revendications

1. Utilisation d'un ou plusieurs agent(s) antidiabétique(s) choisi(s) parmi le carbutamide, le gliquidone, le glisentide, le glisolamide, le glisoxepide, le glycyclamide, et le glibornuride, pour fabriquer un médicament destiné à améliorer la cicatrisation de plaies ou de lésions par voie topique le(s)dit(s) agent(s) antidiabétique(s) étant le(s) seul(s) principe(s) actif(s) dans le médicament.

2. Utilisation selon la revendication 1 pour fabriquer un médicament ayant un effet cicatrisant sous une forme pharmaceutique à usage topique destinée à être appliquée sur la peau.

3. Utilisation selon l'une quelconque des revendications précédentes pour fabriquer un médicament ayant un effet cicatrisant sur les plaies des sujets diabétiques.

## Claims

1. Use of one or more antidiabetic agent(s) selected from carbutamide, gliquidone, glisentide, glisolamide, glisoxepide, glycyclamide and glibornuride for producing a medicament intended to improve the healing of wounds, sores or lesions by topical application, the said antidiabetic agent being the sole active principle in the medicament.

2. Use according to claim 1 for producing a medicament having a healing effect in a pharmaceutical form for topical use intended to be applied to the skin.

3. Use according to any one of the preceding claims for producing a medicament having a healing effect on wounds or sores of diabetics.

## Patentansprüche

1. Verwendung eines oder mehrerer Antidiabetikamittel, ausgewählt aus Carbutamid, Gliquidon, Glisentid, Glisolamid, Glisoxepid, Glycyclamid und Glibornurid, zur Herstellung eines Medikaments zur Verbesserung der Vernarbung von Wunden oder Läsionen über topische Anwendung, wobei das (die) antidiabetische(n) Mittel den (die) einzigen Aktivstoff(e) in dem Medikament darstellt (darstellen).

2. Verwendung gemäß Anspruch 1 zur Herstellung eines Medikaments mit einer vernarbenden Wirkung in einer pharmazeutischen Dosierform zur topischen Anwendung auf der Haut.

3. Verwendung gemäß einem der obigen Ansprüche zur Herstellung eines Medikaments mit einer vernarbenden Wirkung auf den Wunden von Diabetespatienten.
